# EUROPÄISCHE PATENTSCHRIFT

(11) **EP 0 469 444 B1**
(45) Veröffentlichungstag und Bekanntmachung des Hinweises auf die Patenterteilung: **04.06.1997**
(21) Anmeldenummer: 91112388.3
(22) Anmeldetag: 24.07.1991
(51) Int. Cl.: G01N 1/00, B01L 3/02, G01N 35/00

(54) **Verfahren und Vorrichtung zum dosierten Zuführen einer biochemischen Analyseflüssigkeit auf ein Target**
Method and apparatus for disparsing biochemical reagents onto a target in precisely controlled volumes
Procédé et dispositif pour appliquer des doses de réactifs biochimiques sur un support

(30) Priorität: 02.08.1990 DE 4024545
(43) Veröffentlichungstag der Anmeldung: 05.02.1992
(73) Patentinhaber: BOEHRINGER MANNHEIM GMBH, 68305 Mannheim (DE)
(72) Erfinder: Deeg, Rolf, Dr. rer. nat., W-8139 Bernried (DE); Maurer, Eberhard, Dr. rer. nat., W-8120 Weilheim (DE); Babiel, Reiner, Dr. rer. nat., W-8121 Eberfing (DE); Klose, Sigmar, Dr. phil., W-8131 Berg 2 (DE); Köpfer, Bernhard, W-8132 Tutzing (DE)
(74) Vertreter: Pfeifer, Hans-Peter, Dr., Dr. H.-P. Pfeifer Dr. P. Jany, Patentanwälte

(56) Entgegenhaltungen:
- EP-A- 0 119 573
- EP-A- 0 268 237
- FR-A- 2 355 290
- GB-A- 1 218 749
- US-A- 4 376 945

## Beschreibung

Die Erfindung betrifft ein Verfahren zum dosierten Zuführen einer biochemische Makromoleküle enthaltenden Analyseflüssigkeit auf ein Target, bei dem diese Flüssigkeit in kleinen Quanten stoßweise durch eine Düse auf das Target ausgestoßen wird sowie eine entsprechende Vorrichtung.

In der klinischen Chemie stellt sich vielfach die Aufgabe, eine Analyseflüssigkeit auf ein Target dosiert zu applizieren. Die Flüssigkeit kann beispielsweise eine Probenflüssigkeit, insbesondere Blut oder Serum, eine Reagenzflüssigkeit oder eine Kalibrationsflüssigkeit sein. In aller Regel enthalten diese Flüssigkeiten Proteine oder andere an biochemischen Prozessen beteiligte Makromoleküle.

Das Target, dem die Flüssigkeit zugeführt werden soll, kann ein Reaktionsgefäß sein, wobei in Analyseautomaten heute überwiegend sehr kleine Reaktionsgefäße aus Kunststoff verwendet werden. Ein anderes Beispiel sind die in der Mikrobiologie vielfach verwendeten Mikrotiterplatten. Ein für die Erfindung besonders wichtiger Fall ist die Applikation der Analyseflüssigkeit auf einem Analyseelement (vielfach auch als "Testträger" und in der angelsächsischen Literatur als "solid state analysis element" bezeichnet). Dieser Begriff umfaßt im Sinne der vorliegenden Erfindung sowohl diskrete Analyseelemente als auch Bänder, Streifen oder andere Formen von kontinuierlichen Analyseelementen, die an einer Dosierstation, an der die Analyseflüssigkeit appliziert wird, kontinuierlich vorbeigeführt werden können.

Traditionell wurden zum Zuführen von Analyseflüssigkeiten in Analyseautomaten verschiedene Formen von Kolben-Zylinderkonstruktionen (Dispensoren und Dilutoren) verwendet. Auf Analyseelemente wurden Reagenzien überwiegend in der Weise appliziert, daß eine Trägermatrix, beispielsweise aus Papier, in eine Reagenzflüssigkeit eingetaucht wurde oder es wurde in einem schichtbildenden Verfahren ein Reagenzfilm aus einer filmbildenden Polymere enthaltenden Flüssigkeit erzeugt. Soweit gezielt auf einer Basisschicht eine räumlich abgegrenzte Reagenzfläche erzeugt werden sollte, wurde hierzu die Verwendung verschiedener Drucktechniken vorgeschlagen.

Die EP-A-119 573 und die EP-A-268 237 (US-A-4 877 745) befassen sich mit Verfahren und Vorrichtungen der eingangs bezeichneten Art. Ihre Technik basiert auf der ursprünglich für Computerdrucker (Tintenstrahldrucker) entwickelten Ink-Jet-Technologie. Beide Schriften enthalten nähere Erläuterungen zum vorbekannten Stand der Technik, auf die hier Bezug genommen wird.

Die EP-A-119 573 befaßt sich insbesondere mit dem Problem, ein kostengünstiges "Pumpenelement" zur Verfügung zu stellen, welches als disposibles (einmal verwendbares) Bauteil ausgebildet ist. Die Düsenkammer wird dabei im wesentlichen durch einen Abschnitt eines elastisches Schlauches gebildet, welcher Teil des Pumpenelementes ist. Gegen dessen Seitenfläche ist eine elektromagnetisch bewegte Zylinderstange gerichtet, die jeweils gegen den Schlauch bewegt wird, wenn ein Tropfen ausgestoßen werden soll.

In der EP-A-268 237 ist eine Vorrichtung beschrieben, bei der die Düsenkammer aus einem Rohrstück besteht, welches von einem koaxialen, ebenfalls rohrförmig ausgebildeten piezoelektrischen Betätigungselement umschlossen ist und komprimiert wird, wenn ein Tropfen ausgestoßen werden soll.

Der Erfindung liegt die Aufgabe zugrunde, ein Verfahren oder eine Vorrichtung zum Applizieren von Mikroquanten biochemischer Analyseflüssigkeiten zur Verfügung zu stellen, welches im Vergleich zu den vorbekannten Verfahren konstruktiv weniger aufwendig ist und eine hochgenaue Dosierung sehr kleiner Quanten (weniger als 1 µl) mit hoher Frequenz (mehr als 1000 Hz) ermöglicht.

Die Aufgabe wird bei einem Verfahren der eingangs bezeichneten Art dadurch gelöst, daß ein Teilvolumen der Flüssigkeit in der Düsenkammer jeweils kurzfristig verdampft und expandiert wird, um ein Quantum der Flüssigkeit durch die Düse auszustoßen.

Die dem erfindungsgemäßen Verfahren zugrundeliegende Technologie ist von Computerdruckern bekannt. Sie wird dort als "Bubble-Jet-Technik" bezeichnet und ist z.B. aus US-A-4 376 945 bekannt. Im Rahmen der vorliegenden Erfindung wurde überraschenderweise festgestellt, daß sich diese Drucktechnik auf das Applizieren von Analyseflüssigkeiten übertragen läßt.

Die Verwendung dieser Technik für Analyseflüssigkeiten erweist sich als außerordentlich vorteilhaft. Es ist insbesondere möglich, auf wirtschaftliche Weise disposible Düseneinheiten herzustellen, welche die Analyseflüssigkeit (vor allem Reagenzien oder Kalibrationsflüssigkeiten) in vorgepackter Form enthalten. Dadurch werden bedeutende Vereinfachungen und Verbesserungen auf dem Gebiet der automatischen Analyse möglich, wie im folgenden noch näher erläutert wird.

Im Vergleich zu der EP-A-119 573, in der die Möglichkeit von disposiblen "Pumpelementen" mit vorgepackten Reagenzien bereits angesprochen wird, zeichnet sich die Lösung gemäß der vorliegenden Erfindung vor allem dadurch aus, daß keinerlei mechanisch bewegliche Teile erforderlich sind. Dies führt zu einer erhöhten Zuverlässigkeit. Außerdem können sehr kleine Flüssigkeitsquanten mit vergleichsweise hoher Frequenz hergestellt werden.

Im Vergleich zu der EP-A-268 237 wird eine wesentliche konstruktive Vereinfachung erzielt. Die Herstellungskosten sind erheblich niedriger. Die bei dem piezoelektrischen Verfahren erforderliche Reinigung des Düsenkanals entfällt.

Die Tatsache, daß die Bubble-Jet-Technik trotz dieser bedeutenden Vorzüge bisher noch nicht für das dosierte Applizieren biochemischer Analyseflüssigkeiten vorgeschlagen wurde, dürfte darauf zurückzuführen sein, daß diese Technik notwendigerweise bedingt, daß die Analyseflüssigkeit sehr stark erhitzt wird. Es war daher zu befürchten, daß die in der Flüssigkeit enthaltenen Makromoleküle, insbesondere Eiweißsubstanzen, in ihrer Funktion irreversibel geschädigt werden oder daß es zu Denaturierungen oder Aggregatbildungen kommt, die zu einer Verstopfung der Düsen führen. Besonders empfindlich gegen starke Erhitzung sind Enzyme. Überraschenderweise wurde im Rahmen der vorliegenden Erfindung jedoch festgestellt, daß die mit dem Bubble-Jet-Verfahren verbundene Belastung der Analyseflüssigkeit nicht zu einer praktisch bedeutsamen Beschädigung der darin enthaltenen Makromoleküle oder zu Dosierproblemen führt. So haben beispielsweise Vergleichsversuche, bei denen die enzymatische Aktivität in einer bestimmten Lösungsmenge bestimmt und anschließend diese Lösung mit dem erfindungsgemäßen Verfahren verarbeitet wurde, zu dem Ergebnis geführt, daß die ursprüngliche Aktivität nach der Applikation auf ein Target zu über 90% wiedergefunden wurde.

Die Erprobung verschiedener Analyseflüssigkeiten hat überraschenderweise zu dem Ergebnis geführt, daß in einem verhältnismäßig breiten Vikositätsbereich (etwa zwischen 1 centistoks und mehr als 10 centistoks) gearbeitet werden kann. Dies ist ein besonderer Vorteil gegenüber den vorbekannten Ink-Jet-Applikationen von Analyseflüssigkeiten, weil dort ein sehr viel engerer Viskositätsbereich eingehalten werden muß. Auch bezüglich der Oberflächenspannung, die durch Zugabe von Detergenzien oder wahlgeeigneter Lösungsmittel beeinflußt werden kann, hat sich das erfindungsgemäße Verfahren überraschenderweise als relativ unkritisch im Vergleich zu der bekannten Technik erwiesen.

Die Erfindung wird im folgenden anhand eines in den Figuren schematisch dargestellten Ausführungsbeispiels näher erläutert; es zeigen:
- Fig. 1: eine Prinzipdarstellung -teilweise als Blockschaltbild- einer Vorrichtung zur Herstellung von Analyseelementen,
- Fig. 2: einen auf der Erfindung basierenden Analyseautomat in einer perspektivischen Prinzipdarstellung,
- Fig. 3: eine Aufsicht auf einen Reagenzbereich eines Analyseelementes,
- Fig. 4: zwei Eichkurven zu Beispiel 4.

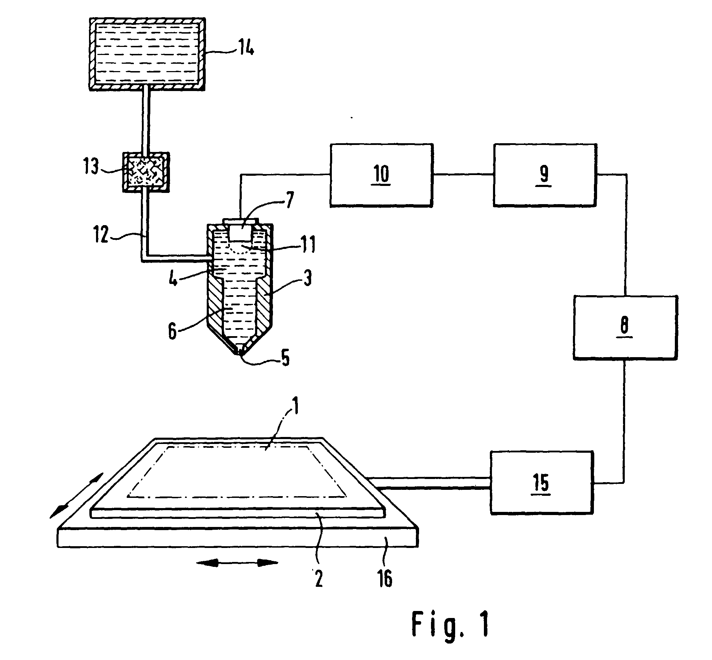

Fig. 1 zeigt, wie Reagenzflüssigkeiten als vorbestimmtes Muster von Kompartimenten auf den Reagenzbereich 1 eines Analyseelementes 2 appliziert werden können. Der Begriff "Kompartiment" (englisch: compartment) bezeichnet dabei einen abgegrenzten Teilbereich. Über dem Reagenzbereich 1 ist ein insgesamt mit 3 bezeichneter Düsenkopf mit einer Düsenkammer 4 und einer Düse 5 angeordnet. In der Düsenkammer 4 befindet sich ein Heizelement 7, welches in thermischem Kontakt zu in der Düsenkammer 4 enthaltener Analyseflüssigkeit 6 steht. Von einer Steuereinheit 8 gesteuert wird über einen Impulsgeber 9 und einen Verstärker 10 jeweils ein Stromimpuls an das Heizelement 7 angelegt, wenn ein Quantum Analyseflüssigkeit 6 aus der Düse 5 ausgestoßen werden soll. Dabei bildet sich sehr schnell (innerhalb von ca. 200 µsec) eine Dampfblase 11. Durch deren Expansion wird ein Flüssigkeitstropfen aus der Düse 5 ausgestoßen. Die Düsenkammer 4 ist über eine Leitung 12 mit Filter 13 mit einem Vorratsbehälter 14 für Analyseflüssigkeit 6 verbunden. Düsenkopf 3, Filter 13 und Vorratsbehälter 14 können in einer disposiblen Cartridge ("Düseneinheit") untergebracht sein.

Das Analyseelement 2 läßt sich mit Hilfe eines ebenfalls von der Steuereinheit 8 gesteuerten X-Y-Antriebs 15 und eines Positioniertisches 16 in beide Flächenrichtungen des Reagenzbereiches 1 so positionieren, daß nacheinander aus der Düse 5 ausgestoßene Reagenzflüssigkeitsquanten ein vorbestimmtes Muster bilden. Selbstverständlich kann alternativ oder zusätzlich auch der Düsenkopf 3 entsprechend bewegt werden.

In Fig. 1 ist ein Düsenkopf 3 mit nur einem Heizelement 7 und einer Düse 5 dargestellt. Vorteilhafterweise können jedoch die für Tintenstrahl-Druckverfahren gebräuchlichen Düsenköpfe mit einer Mehrzahl von Düsen (meist 9, 12 oder 24 Düsen) verwendet werden. Dadurch kann die erforderliche Bewegung des Analyseelements 2 bei der Erzeugung eines vorbestimmten Musters reduziert und die Dosierleistung erhöht werden. Insbesondere ist es möglich, mit einer Relativbewegung des Analyseelements 2 bezüglich des Düsenkopfes 3 in nur einer Raumrichtung ein zweidimensionales Muster der Analyseflüssigkeit 6 auf der Reagenzfläche 1 zu erzeugen. Bei Verwendung eines Düsenkopfes 3 mit mehreren Kanälen ist selbstverständlich der Impulsgeber 9 und der Verstärker 10 entsprechend mehrkanalig ausgelegt.

Abgesehen von den hier beschriebenen Besonderheiten können die für die Bubble-Jet-Technik bekannten konstruktiven Elemente bei der Erfindung verwendet werden. Es ist deshalb nicht erforderlich, auf konstruktive Einzelheiten der Vorrichtung, insbesondere der Düsenkammer, der Düse oder der Heizelemente einzugehen. Diese Angaben können der Literatur über Bubble-Jet-Tintenstrahldrucker entnommen werden.

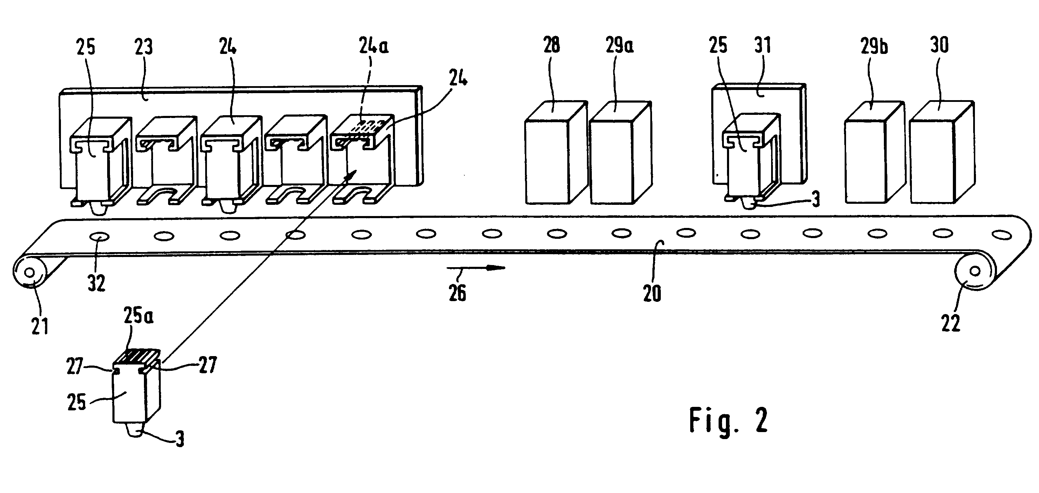

Wie oben dargelegt besteht ein besonderer Vorteil der Erfindung darin, daß eine so kostengünstige Herstellung einer Düseneinheit möglich ist, daß sie als disposibles Element gestaltet sein kann, welches einen Vorrat an Analyseflüssigkeit benutzungsfertig (herstellerseitig vorgepackt) enthält. Dadurch entfallen aufwendige habungsschritte bei der Durchführung von Analysen mit entsprechenden Geräten. So ist es möglich, mit verhältnismäßig geringem konstruktivem Aufwand außerordentlich vielseitig einsetzbare und einfach bedienbare Analysesysteme (bestehend aus Gerät und spezifisch darauf abgestimmten Reagenzien) zur Verfügung zu stellen. Ein solches System ist beispielhaft in Fig. 2 dargestellt.

Bei dem in Fig. 2 dargestellten Gerät wird als Analyseelement ein Band 20 eingesetzt, welches aus einem geeigneten Reagenz-Trägermaterial, beispielsweise Papier oder einer Kunststoffolie besteht. Es wird schrittweise von einer Anfangsrolle 21 zu einer Endrolle 22 transportiert. Über dem Band 20 sind in einer Reagenzdosierstation 23 mehrere geräteseitige Halterungen 24 angeordnet, die mit Halterungselementen 27 an den Düseneinheiten 25 zusammenwirken, um diese auswechselbar an definierten Positionen über dem Band 20 zu positionieren. An den Halterungen 24 und an den Düseneinheiten 25 sind jeweils elektrische Kontakte 24a, 25a vorgesehen, die eine elektrische Verbindung zwischen dem Gerät und einer in eine Halterung 24 eingesetzten Düseneinheit ermöglichen.

In Transportrichtung des Bandes 20 (Pfeil 26) hinter der Reagenzdosierstation 23 befinden sich weitere Bearbeitungseinheiten, im dargestellten Fall eine Probendosiereinheit 28, zwei Wascheinheiten 29a, 29b, eine weitere Reagenzdosierstation 31 und eine Meßeinheit 30.

Das Analyseverfahren beginnt damit, daß Analyseflüssigkeiten, insbesondere Reagenzien durch eine oder mehrere der Düseneinheiten 25 der Reagenzdosierstation 23 auf das Band 20 appliziert werden, die auf dem Band Reagenzbereiche 32 bilden. Um die gewünschte Flächenausdehnung der Reagenzbereiche 32 senkrecht zu der Transportrichtung 26 zu gewährleisten, haben die Düseneinheiten 25 mehrere nebeneinanderangeordnete Düsen in ihrem Düsenkopf 3. Alternativ oder zusätzlich können sie mit einer nicht dargestellten Mechanik quer zur Transportrichtung 26 des Bandes 20 beweglich sein.

Durch die Probendosiereinheit 28 wird eine Probe zugegeben. Die Wascheinheiten 29a und 29b erlauben erforderlichenfalls die Durchführung von Waschschritten. Die Reagenzdosierstation 31 erlaubt die Dosierung eines weiteren Reagenzes. Die Meßeinheit 30 dient dazu, eine für die Analyse charakteristische physikalische Meßgröße, beispielsweise die optische Reflexion oder Fluoreszenz bei einer bestimmten Meßwellenlänge zu messen.

Auf nähere Einzelheiten möglicher Verfahrensführungen wird hier nicht eingegangen. Die Erfindung kann bei einer Vielzahl verschiedener Verfahren (z.B. homogene und heterogene Immunoassays, enzymatische Bestimmungen etc.) eingesetzt werden, bei denen ein Analyseelement nach der Zuführung der Reagenzflüssigkeit in einem kontinuierlichen Prozeß zu einer Probenzugabestation transportiert, eine Probe mit der Reaktionsfläche kontaktiert und eine in Folge der Reaktion von Probe und Reagenz eintretende physikalisch meßbare Veränderung gemessen wird. Diese Verfahrensweise wurde bereits früher, insbesondere in der US-A-3 526 480 vorgeschlagen, auf welche hier Bezug genommen wird.

Entscheidend im Zusammenhang mit der vorliegenden Erfindung ist, daß bezüglich der Reagenzzugabe eine sehr einfache und flexible Anpassung an die Erfordernisse der jeweiligen Analyse möglich ist. So kann das Gerät durch einfaches Auswechseln der Düseneinheiten 25 auf verschiedene Analysen, die mit verschiedenen Reagenzien arbeiten, umgestellt werden, ohne daß Reagenzbehälter ausgetauscht und die üblicherweise verwendeten Zuführungsschläuche sowie Dosierungssysteme gespült werden müssen. Die Anordnung mehrerer Düsenelementhalterungen entlang der Transportbahn eines schrittweise in einem kontinuierlichen Prozeß transportierten Analyseelementes ermöglicht es, einerseits mehrere verschiedene Reagenzien zu verschiedenen Zeitpunkten zu dosieren und andererseits auch für ein einzelnes Reagenz die Zeit, die zwischen der Applikation auf dem Analyseelement und der Probenzugabe verstreicht, in einfacher Weise den jeweiligen Erfordernissen anzupassen.

Ein derartig einfacher Aufbau war bisher nur mit Hilfe vorgefertigter Analyseelemente, wie sie insbesondere in der äußeren Form von Teststreifen oder als sogenannte "analysis slides" gebräuchlich sind, möglich. Dies erforderte jedoch eine aufwendige Transportmechanik für die Analyseelemente. Darüber hinaus mußten die Analyseelemente zwischen Herstellung und Verwendung über längere Zeit gelagert werden. Damit ist in Anbetracht der problematischen Lagerstabilität solcher Analyseelemente ein hoher Aufwand verbunden. Die Erfindung ermöglicht es, das Analyseelement mit einer die gewünschte Reagenzkombination enthaltenden Reagenzfläche unmittelbar vor der Verwendung (d.h. vor der Zugabe der Probe) auf einfache Weise frisch herzustellen.

Das kontinuierlich transportierte Analyseelement muß nicht notwendigerweise bandförmig gestaltet sein. Je nach den Erfordernissen der Analyse könnten auch kleine Reaktionsgefäße, beispielsweise in Form von miteinander verketteten flachen Schalen aus Kunststoff oder andere kontinuierlich transportierbare Reagenzträger verwendet werden.

Die Erfindung kann vorteilhaft für die Applikation der verschiedensten in der klinischen Chemie üblichen Reagenzien auf einem festen Träger verwendet werden. Beispielsweise können Enzyme, Substrate oder andere lösliche Reaktionsbestandteile so auf den Träger aufgebracht werden, daß sie leicht eluierbar sind, um in flüssiger Phase zu reagieren. Aber auch für die Applikation von trägerfixierten Reagenzien (insbesondere Antikörper, Antigene etc.) kann die Erfindung vorteilhaft eingesetzt werden. Schließlich kann es zweckmäßig sein, auf der Trägeroberfläche, auf die die Applikation nach dem erfindungsgemäßen Verfahren erfolgt, bereits zuvor nach anderen Verfahren Reaktionsbestandteile aufzubringen. So kann beispielsweise ein Trägermaterial großflächig mit einer Streptavidin enthaltenden Oberflächenbeschichtung versehen sein, auf welche selektiv und gezielt biotinylierte Reagenzien appliziert werden, welche über die Biotin-Streptavidin-Bindung an den Träger gebunden werden. Nähere Einzelheiten sind in der EP0 469 445 A1 beschrieben, auf welche hier Bezug genommen wird.

Wenn gemäß dem erfindungsgemäßen Verfahren eine Mehrzahl verschiedener Reagenzflüssigkeiten auf einen Reagenzbereich aufgebracht wird, ist es vorteilhaft, das Applikationsmuster so zu wählen, daß die von Quanten verschiedener Reagenzflüssigkeiten erzeugten Kompartimente sich gegenseitig nicht kontaktieren. Dies gilt insbesondere, wenn die in den Flüssigkeiten enthaltenen Reagenzien miteinander (zumindest im flüssigen Zustand) interferieren. Vorzugsweise bilden dabei die Quanten verschiedener Reagenzflüssigkeiten ein Muster von alternierenden Kompartimenten, wobei sie dicht beieinander, aber räumlich getrennt, vorliegen. Auch insoweit wird auf die genannte gleichzeitig eingereichte Anmeldung Bezug genommen.

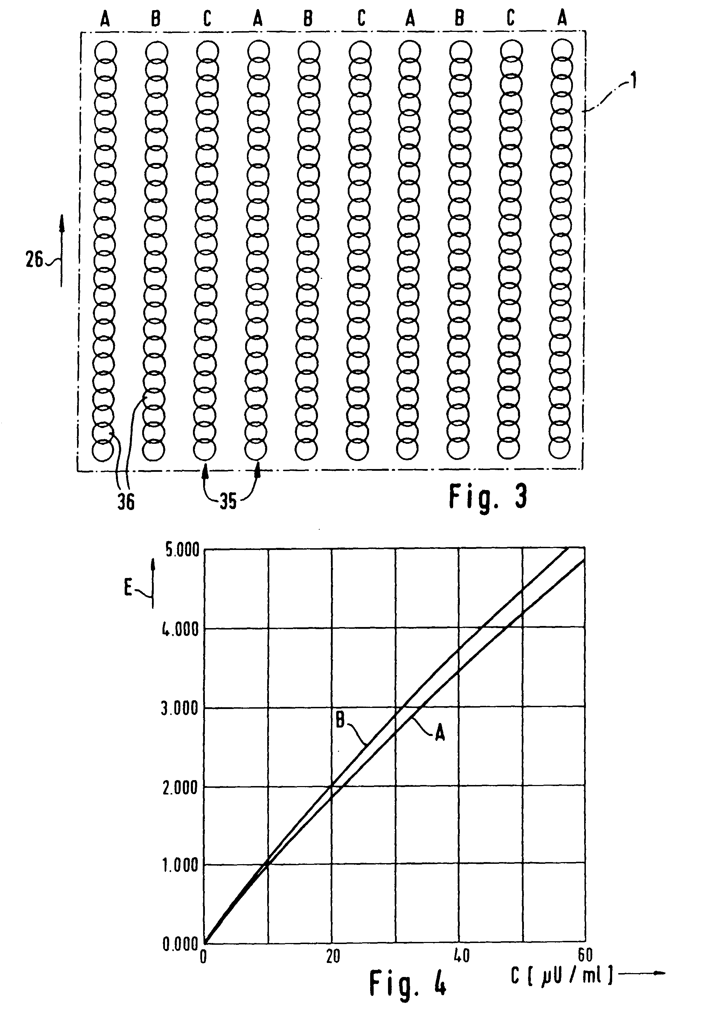

Ein solches Kompartimentmuster ist in Fig. 3 dargestellt.

Die Quanten der Reagenzflüssigkeit bilden im dargestellten Beispiel eine Mehrzahl von reihenförmigen Kompartimenten 35, die jeweils aus vielen dicht beieinander geordneten Flächenelementen ("Dots") 36 bestehen, die jeweils von einem Quantum der Reagenzflüssigkeit erzeugt sind. Für den Fall, daß der Träger in einem kontinuierlichen Prozeß, wie er in Fig. 2 dargestellt ist, mit der Reagenzflüssigkeit beschickt wird, können die verschiedenen Kompartimente der gleichen Reagenzzusammensetzung zweckmäßigerweise aus verschiedenen Düsen der gleichen Düseneinheit 25 ausgestoßen werden. Die Transportrichtung verläuft in diesem Fall parallel zu den Reihen (Pfeil 26).

Die am oberen Rand der Fig. 3 angegebenen Buchstaben bezeichnen drei verschiedene Sätze A,B,C, von Kompartimenten 35, wobei die Kompartimente 35 des gleichen Satzes die gleichen Reagenzien enthalten, während die Reagenzien verschiedener Sätze sich in ihrer chemischen Zusammensetzung unterscheiden.

Statt der dargestellten alternierenden Reihen können auch andere Formen von Flächenelementen verwendet werden. Insbesondere kann ein Punktmuster, welches aus sich gegenseitig nicht überlappenden Dots besteht, (so daß jeweils ein Dot ein Kompartiment bildet), zweckmäßig sein.

Im dargestellten Beispielsfall haben die Dots innerhalb der Kompartimente einen Mittenabstand von wenig mehr als 0,1 mm. Der Mittenabstand der Kompartimente voneinander beträgt ca. 0,26 mm. Dabei wurde von den drucktechnisch möglichen Kompartimenten nur jedes zweite verwendet. Grundsätzlich ist ein noch engerer Abstand der Flächenelemente möglich.

Im Rahmen der vorliegenden Erfindung wurde festgestellt, daß eine solch enge mit dem erfindungsgemäßen Verfahren hergestellte Anordnung von aus verschiedenen Reagenzflüssigkeiten bestehenden Flächenelementen in vielen Fällen vorteilhafte Analyseverfahren ermöglicht. Soweit bisher verschiedene miteinander unverträgliche Reagenzien in einem Analyseelement verwendet wurden, wurden diese in aller Regel in verschiedene Schichten eines mehrschichtigen Analyseelements integriert, wobei diese Schichten entweder separat hergestellt und anschließend zusammengefügt wurden oder nacheinander in einem schichtbildenden Verfahren auf eine Basisschicht aufgetragen wurden. Zwar wurde in der DE-C2-27 29 333 bereits vorgeschlagen, miteinander unverträgliche Reagenzien im Siebdruckverfahren auf eine Fläche derartig aufzutragen, daß die Flächenelemente alternieren. Dieses bekannte Verfahren ist jedoch sehr aufwendig und erfordert lange Reaktionszeiten. In Verbindung mit dem erfindungsgemäßen Verfahren ist auf einfache Weise ein so enger Abstand der Flächenelemente möglich, daß sich nach Aufgabe der Probe die verschiedenen Reagenzien schnell praktisch vollständig mischen und homogen reagieren.

Die folgenden Beispiele dienen der weiteren Erläuterung der Erfindung.

### Beispiel 1:

Die Tinte eines nach dem Bubble-Jet-Prinzip arbeitenden Tintenstrahl-Druckkopfes (Hewlett-Packard Typ Quiet Jet plus) wird gegen eine Tartrazin-Farbstofflösung ausgetauscht.

Daten des Druckkopfes:
- 12 Düsen in Reihe angeordnet
- Tropfendurchmesser ca. 75 µm
- kleinstes dosierbares Quantum (1 Tropfen) 230 Pikoliter
- maximale Druckdichte pro Druckschritt: 192 x 192 Tropfen/inch²

Dieser Druckkopf ist zusammen mit einem Tintenvorratsbehälter in einer entnehmbaren Cartridge untergebracht. Die Steuerung des Druckkopfes erfolgt über einen Personal Computer mit Hilfe eines Basic-Steuerprogramms.

Volumina zwischen 0,1 und 5 µl der Farbstofflösung (40 mg/ml Tartrazin in 40 mM Natriumphosphat-Puffer (NaPP) pH 7,4) wurden in Reagenzröhrchen, die mit 2 ml destilliertem Wasser gefüllt waren, dosiert. Anschließend wurde in einem kommerziell erhältlichen Analysegerät (ES 22 der Boehringer Mannheim GmbH) sorgfältig gemischt und die Extinktion bei 405 nm gemessen. Die Präzision der Dosierung wurde anhand von 15-fach Bestimmungen errechnet. Die Ergebnisse sind in Tabelle 1 dargestellt.

### Beispiel 2:

Bei diesem Beispiel wurde analog zu Beispiel 1 eine Lösung von 0,5 mg/ml des Enzyms Peroxidase in 40 mM NaPP pH 7,4, 3 Gewichts-% Polyvinylpyrrolidon, 0,01 Gewichts-% Triton-X-100 dosiert. In den Röhrchen war in diesem Fall jeweils 2 ml ABTS^{R}-Substratlösung (1,9 mmol/l 2,2'-Azino-di-[3-äthyl-benzthiazolin-sulfonsäure(6)] -diammoniumsalz; 100 mmol/l Phosphat-Citrat-Puffer pH 4,4; 2,2 mmol/l Natriumperborat) vorgelegt. Die dosierten Volumina lagen zwischen 0,23 und 80 nl. Die Bestimmung der Extinktionen und der Dosierpräzisionen erfolgten analog zu Beispiel 1.

Die Ergebnisse von Beispiel 2 sind in der Tabelle 2 dargestellt.

**Tabelle 1 :**

| | | | | | |
|---|---|---|---|---|---|
| VOLUMEN (µl) | 0,1 | 0,2 | 0,6 | 2 | 5 |
| VK (%) | 1,4 | 0,96 | 0,63 | 0,57 | 0,65 |

**Tabelle 2 :**

| | | | | | |
|---|---|---|---|---|---|
| VOLUMEN (nl) | 0,23 | 0,46 | 1 | 20 | 80 |
| VK (%) | 7,77 | 4,95 | 3,84 | 4,1 | 1,98 |

In den Tabellen ist jeweils das Soll-Volumen und der Variationskoeffizient VK angegeben. Die VK-Werte, die sich auf jeweils 15 Messungen beziehen, zeigen, daß die Präzision der Dosierung (in Relation zu den sehr geringen Volumina) ausgezeichnet ist.

### Beispiel 3:

Mit einem Druckkopf entsprechend den Beispielen 1 und 2 wird eine Reagenzflüssigkeit mit folgender Zusammensetzung verarbeitet:

| | |
|---|---|
| 100 mM | Tris/HCl pH 7,9 |
| 15 mM | Tribromhydroxybenzoesäure |
| 5 mM | 3-Methyl-2-benzo-(2'-sulfo)-thiazolinon-hydrazon |
| 50 U/ml | Sarcosin-Oxidase |
| 10 U/ml | Peroxidase |

Die Beschichtungslösung wird mit maximaler Druckdichte (192x192 Tropfen/inch²) auf ein saugfähiges Papier aufgebracht, wobei sechs getrennte Reagenzflächen erzeugt werden. Der Auftrag wird dreimal wiederholt, wobei die insgesamt applizierte Menge an Reagenzflüssigkeit etwa 3,9 µl/cm² beträgt. Anschließend wird bei Raumtemperatur getrocknet (ca. 30 min.) oder sofort die Probe aufgetragen.

Trägt man auf die so erhaltenen sechs Reagenzflächen jeweils 1 µl Probe mit unterschiedlichen Sarcosin-Konzentrationen zwischen 0 und 100 mM auf, so ergibt sich nach etwa 1 min ein gut abgestufter Farbumschlag, der in üblicher Weise kalibriert und reflexionsphotometrisch vermessen werden kann. Die visuelle Detektionsgrenze liegt bei ca. 10 ng Sarcosin/µl.

Das Beispiel zeigt, daß erfindungsgemäß auch ein mit relativ hohen Reagenzmengen arbeitendes Analyseelement für einen enzymatischen Test auf einfache Weise hergestellt werden kann. Gegenüber konventionellen Tests ergibt sich eine bedeutende Ersparnis an Reagenz und es kann mit einer sehr kleinen Probenmenge gearbeitet werden. Die niedrige Nachweisgrenze zeigt, daß die Enzymaktivität praktisch vollständig erhalten geblieben ist.

### Beispiel 4:

Eine Analyse des Schilddrüsen-Hormons TSH wird einerseits mit einem konventionellen Immun-Analysesystem (Enzymunsystem ES 22 der Boehringer Mannheim GmbH, Versuch A) und andererseits mit einem erfindungsgemäß modifizierten System (Versuch B) durchgeführt. Die einzelnen Schritte laufen bei Versuch B wie folgt ab:
a) Es werden mit Streptavidin beschichtete Polystyrol-Tubes (Herstellung nach EP-A-0344578) verwendet. In diese werden jeweils 100 µl Probe bzw. Standard dosiert.
b) Mit Hilfe eines Druckkopfes wie bei den Beispielen 1-3 wird 10 µl einer über einen 0,8 µm Filter filtrierten Konjugatlösung appliziert. Die Konjugatlösung enthält 18 U/ml eines Konjugates aus einem gegen TSH gerichteten monoklonalen Antikörper (ECACC 87122202) und Peroxidase in 80 mM Natriumphosphat-Puffer (NaPP) pH 7,4.
c) Über die Dosiereinheit des genannten Systems wird 1 min nach Zugabe des Konjugates 1ml Inkubationspuffer (80 mM NaPP pH 7,4 mit 1250 µg/ml eines biotinylierten monoklonalen Antikörpers gegen TSH (ECACC 87122201), 2g/l Rinder-Serumalbumin, 1 g/l Rinder-IgG) zudosiert. (Die Biotinylierung des Antikörpers erfolgte gemäß JACS 100 (1978,3585-3590) durch Umsetzung mit N-hydroxysuccinimid-Biotin im Verhältnis 10:1).
d) Anschließend wird für 60 min inkubiert.
e) Mit der Dosiereinheit des verwendeten Systems werden fünf Waschschritte, jeweils bestehend aus Absaugen der Reagenzlösung und Zudosierung von Leitungswasser durchgeführt.
f) Wiederum über die Dosiereinheit wird 1 ml Enzymun-ABTS^{R}-Substratlösung zudosiert.
g) Es wird für 30 min inkubiert.
h) Die Extinktion der Substratlösung wird mit Hilfe der photometrischen Meßeinrichtung des Systems bei 405 nm vermessen.

Bei dem konventionellen Vergleich (Versuch A) werden die Schritte b und c zusammengefaßt. Das Konjugat aus Schritt b ist in diesem Fall in einer Konzentration von 18 U/ml in dem unter Schritt c beschriebenen Inkubationspuffer zugesetzt. 1 ml dieser Gesamtlösung wird über die Dosiereinheit des genannten Systems zudosiert.

Die Eichung erfolgt mit üblichen Standards zwischen 0 und 51,1 µU TSH/ml.

**Tabelle 3**

| Präzision / Wiederfindung von Kontrollseren | Versuch B | Versuch A |
|---|---|---|
| x (µU/ml) | 1,8 | 1,99 |
| VK (%) | 3,1 | 2,9 |
| x (µU/ml) | 5,74 | 6,26 |
| VK (%) | 4,8 | 3,2 |

In Tabelle 3 sind die Ergebnisse für zwei verschiedene Sollwerte (1,9 µU/ml und 6,0 µU/ml) von Kontrollseren mit jeweils 12 Messungen einerseits für den erfindungsgemäßen modifizierten Verfahrensablauf und andererseits für den Vergleichsversuch wiedergegeben. Es werden vergleichbare Ergebnisse hinsichtlich Präzision und Wiederfindung des Sollwertes erreicht.

Fig. 4 zeigt die Eichkurven der Versuche A und B, d.h. die Extinktion E in Abhängigkeit von der Konzentration von Standardlösungen. Die Tatsache, daß beide Kurven sehr eng übereinstimmen, ist ein weiterer Beleg dafür, daß die Eigenschaften proteinhaltiger Lösungen, im vorliegenden Fall des Antikörper-Enzymkonjugates, durch die Applikation mit dem erfindungsgemäßen Verfahren praktisch nicht verändert werden.

Dieses Ergebnis belegt in besonderem Maße die Eignung des erfindungsgemäßen Verfahrens zur Dosierung von sehr kleinen Quanten von Reagenzflüssigkeit, weil der Analyt TSH in einer extrem niedrigen Konzentration gegenwärtig ist.

## Patentansprüche

1. Verfahren zum dosierten Zuführen einer biochemische Makromoleküle enthaltenden Analyseflüssigkeit auf ein Target, bei welchem diese Flüssigkeit in kleinen Quanten stoßweise aus einer Düsenkammer durch eine Düse auf das Target ausgestoßen wird, **dadurch gekennzeichnet,** daß ein Teilvolumen der Flüssigkeit in der Düsenkammer jeweils kurzfristig verdampft und expandiert wird, um ein Quantum der Flüssigkeit durch die Düse auszustoßen.

2. Verfahren nach Anspruch 1, **dadurch gekennzeichnet,** daß die Analyseflüssigkeit eine Reagenzflüssigkeit ist.

3. Verfahren nach Anspruch 1 oder 2, **dadurch gekennzeichnet,** daß die Analyseflüssigkeit ein Protein, insbesondere ein Bindeprotein, ein Enzym, einen Antikörper, ein Antikörper-Enzym-Konjugat, ein Antigen, ein Hapten oder ein Substrat enthält.

4. Verfahren nach einem der vorhergehenden Ansprüche, **dadurch gekennzeichnet,** daß das Target ein Reaktionsgefäß ist, dem die Analyseflüssigkeit zugeführt wird.

5. Verfahren nach einem der Ansprüche 2 oder 3, **dadurch gekennzeichnet,** daß das Target ein Reagenzbereich eines Analyseelementes ist, mehrere Reagenzflüssigkeitsquanten nacheinander ausgestoßen werden und die Düse und das Analyseelement relativ zueinander derartig bewegt werden, daß die von den Reagenzflüssigkeitsquanten in dem Reagenzbereich erzeugten Dots ein vorbestimmtes Muster von Kompartimenten in dem Reagenzbereich bilden.

6. Verfahren nach Anspruch 5, **dadurch gekennzeichnet**, daß das Analyseelement nach der Zuführung der Reagenzflüssigkeit in einem kontinuierlichen Prozeß zu einer Probenzugabestation transportiert, eine Probe mit dem Reagenzbereich kontaktiert und eine infolge der Reaktion von Probe und Reagenz eintretende physikalisch meßbare Veränderung gemessen wird.

7. Verfahren nach Anspruch 5 oder 6, **dadurch gekennzeichnet**, daß mehrere verschiedene Reagenzflüssigkeiten jeweils aus einer gesonderten Düsenkammer durch gesonderte Düsen ausgestoßen werden.

8. Verfahren nach Anspruch 7, **dadurch gekennzeichnet**, daß die von Quanten verschiedener Reagenzflüssigkeiten erzeugten Kompartimente in dem vorbestimmten Muster sich im wesentlichen nicht kontaktieren.

9. Verfahren nach Anspruch 8, **dadurch gekennzeichnet**, daß die Quanten verschiedener Reagenzflüssigkeiten ein Muster von alternierenden Flächenelementen bilden, bei dem Quanten verschiedener Reagenzflüssigkeiten benachbart, aber räumlich getrennt vorliegen.

10. Verfahren nach einem der Ansprüche 5 bis 9, **dadurch gekennzeichnet**, daß ein von einem Quantum Reagenzflüssigkeit erzeugter Dot auf der Reagenzfläche eine Flächenausdehnung von weniger als 2 mm² hat.

11. Vorrichtung zum dosierten Zuführen einer biochemische Makromoleküle enthaltenden Analyseflüssigkeit auf ein Target,
bei der diese Analyseflüssigkeit herstellerseitig vorgepackt in einer mit einer Düse (5) versehenen Düsenkammer eines disposiblen Düsenelementes (25) enthalten ist,
**dadurch gekennzeichnet,** daß
die Düsenkammer (4) ein Heizelement (11) in thermischem Kontakt mit der Flüssigkeit aufweist, durch welches ein Teilvolumen der Analyseflüssigkeit (6) kurzfristig verdampft und expandiert werden kann, um ein Quantum der Analyseflüssigkeit (6) durch die Düse (5) auszustoßen und
außenseitig an dem Düsenelement (25) ein Halterungselement (27) und elektrische Kontakte (25a) vorgesehen sind, um das Düsenelement an ein Analysegerät auswechselbar mechanisch und elektrisch anzuschließen.

## Claims

1. A method for the metered application of an analytical liquid containing biochemical macromolecules to a target, wherein this liquid is ejected in small quantities on to the target through a jet from a jet chamber, **characterized in that** a partial volume of the liquid in the jet chamber is evaporated and expanded for a short time in order to eject each quantity of the liquid through the jet.

2. A method according to Claim 1, **characterized in that** the analytical liquid is a liquid reagent.

3. A method according to Claim 1 or Claim 2,
**characterized in that** the analytical liquid contains a protein, especially a binding protein, an enzyme, an antibody, an antibody-enzyme conjugate, an antigen, a hapten or a substrate.

4. A method according to any one of the preceding claims, **characterized in that** the target is a reaction vessel into which the analytical liquid is introduced.

5. A method according to Claim 2 or Claim 3,
**characterized in that** the target is a reagent domain of an analysis element, several quantities of liquid reagent are ejected successively and the jet and the analysis element are moved relative to one another in such a way that the dots produced by the quantities of liquid reagent in the reagent domain form a predetermined pattern of compartments in the reagent domain.

6. A method according to Claim 5, **characterized in that** the analysis element, after application of the liquid reagent, is conveyed in a continuous process to a sample delivery station, a sample is brought into contact with the reagent domain and a physically measurable change occurring as a consequence of the reaction between sample and reagent is measured.

7. A method according to Claim 5 or Claim 6,
**characterized in that** several different liquid reagents are each ejected from a separate jet chamber through separate jets.

8. A method according to Claim 7, **characterized in that** there is essentially no contact between the compartments produced by quantities of different liquid reagents in the predetermined pattern.

9. A method according to Claim 8, **characterized in that** the quantities of different liquid reagents form a pattern of alternating dots in which quantities of different liquid reagents are adjacent but spatially separated.

10. A method according to any one of Claims 5 to 9,
**characterized in that** a dot produced by a quantity of liquid reagent on the reagent domain has a surface area of less than 2 mm².

11. A device for the metered application of an analytical liquid containing biochemical macromolecules to a target, wherein this analytical liquid is contained in a jet chamber of a disposable jet element (25), provided with a jet (5), prepacked by the manufacturer, **characterized in that** the jet chamber (4) has a heating element (11) in thermal contact with the liquid, by means of which a partial volume of the analytical liquid (6) can be evaporated and expanded for a short time in order to eject a quantity of the analytical liquid (6) through the jet (5), and a fixing element (27) and electrical contacts (25a) are provided on the outside of the jet element (25) for interchangeably connecting the jet element mechanically and electrically to an analyzer.

## Revendications

1. Procédé destiné à amener sur une cible des quantités dosées d'un liquide analytique contenant des macromolécules biochimiques, dans lequel ce liquide est expulsé sur la cible par petits quanta, de façon intermittante, à partir d'une chambre d'injection à travers une buse, caractérisé en ce qu'un volume partiel du liquide est rapidement vaporisé et expansé dans la chambre d'injection pour expulser un quantum de liquide à travers la buse.

2. Procédé selon la revendication 1, caractérisé en ce que le liquide analytique est un réactif liquide.

3. Procédé selon la revendication 1 ou 2, caractérisé en ce que le liquide analytique est une protéine, en particulier une protéine de liaison, une enzyme, un anticorps, un conjugué anticorps-enzyme, un antigène, un haptène ou un substrat.

4. Procédé selon l'une quelconque des revendications précédentes, caractérisé en ce que la cible est un récipient réactionnel, dans lequel le liquide analytique est introduit.

5. Procédé selon l'une des revendications 2 ou 3, caractérisé en ce que la cible est une zone à réactif d'un élément analytique, plusieurs quanta de réactif liquide sont expulsés successivement et la buse ainsi que l'élément analytique sont actionnés l'un par rapport à l'autre de telle façon que les points obtenus dans la zone à réactif à partir des quanta de réactif liquide forment un motif prédéterminé de compartiments dans la zone à réactif.

6. Procédé selon la revendication 5, caractérisé en ce que l'élément analytique est transporté, après l'amenée du réactif liquide, dans un procédé continu, à un poste d'introduction de l'échantillon, un échantillon est mis en contact avec la zone à réactif et une variation physique mesurable, se produisant par suite de la réaction de l'échantillon avec le réactif, est mesurée.

7. Procédé selon la revendication 5 ou 6, caractérisé en ce que plusieurs réactifs liquides différents sont expulsés chacun à partir d'une chambre d'injection particulière par des buses particulières.

8. Procédé selon la revendication 7, caractérisé en ce que les compartiments obtenus à partir des quanta de différents réactifs liquides ne sont pratiquement pas en contact entre eux à l'intérieur du motif prédéterminé.

9. Procédé selon la revendication 8, caractérisé en ce que les quanta de différents réactifs liquides forment un motif d'éléments de surface alternés, les quanta de différents réactifs liquides étant contiguës, mais séparés entre eux dans l'espace.

10. Procédé selon l'une quelconque des revendications 5 à 9, caractérisé en ce qu'un point obtenu à partir d'un quantum de réactif liquide présente, sur la surface à réactif, une superficie inférieure à 2mm².

11. Dispositif pour l'amenée sur une cible, en quantités dosées, d'un liquide analytique contenant des macromolécules biochimiques, dans lequel ce liquide analytique est contenu, sous forme préemballée par le fabricant, dans une chambre d'injection, munie d'une buse (5), d'un élément d'injection (25) jetable,
caractérisé en ce que
la chambre d'injection (4) présente un élément chauffant (11) qui est en contact thermique avec le liquide et qui sert à vaporiser et expanser rapidement un volume partiel du liquide analytique (6), afin d'expulser par la buse (5) un quantum de liquide analytique (6), et à l'extérieur de l'élément d'injection (25), il est prévu un élément de fixation (27) et des contacts électriques (25a) permettant de raccorder l'élément d'injection à un appareil d'analyse par des moyens mécaniques et électriques de façon que l'élément d'injection soit remplaçable.
